Europäisches Patentamt

European Patent Office

Office européen des brevets

(11) Publication number: 0 364 971
A2

# EUROPEAN PATENT APPLICATION

(21) Application number: 89119289.0

(22) Date of filing: 17.10.89

(51) Int. Cl.5: G01N 33/569

(30) Priority: 20.10.88 DE 3835784

(43) Date of publication of application:
25.04.90 Bulletin 90/17

(84) Designated Contracting States:
AT BE CH DE ES FR GB GR IT LI NL SE

(71) Applicant: UNILEVER NV
Burgemeester s'Jacobplein 1 P.O. Box 760
NL-3000 DK Rotterdam(NL)

(84) BE CH DE ES FR GR IT LI NL SE AT

Applicant: UNILEVER PLC
Unilever House Blackfriars
London EC4P 4BQ(GB)

(84) GB

(72) Inventor: Fehrenbach, Franz, Prof. Dr. med
Neubrückerstrasse 75
D-1000 Berlin 28(DE)

(74) Representative: Hagemann, Heinrich, Dr.
Dipl.-Chem. et al
Patentanwälte HAGEMANN & KEHL
Ismaninger Strasse 108 Postfach 860329
D-8000 München 80(DE)

(54) Process for the detection of legionella antigens in body fluids and the like, as well as a test kit for performing the process.

(57) A process is described for the rapid detection of legionella antigens in body fluids, tissue homogenates or other legionella antigen-containing liquids with specific antibodies for the same, accompanied by the formation of a labelled antigen-antibody complex and the detection thereof by means of conventional immunoassays. It is characterized in that the immunoassay is mainly directed at the detection of an in vivo or in vitro processed antigen fraction with an apparent molecular weight of approximately 68 and/or approximately 25 kDa, determined by the SDS-PAGE method. It also describes a test kit for performing the process, which a) contains an antibody conjugate and b) a capture antibody. This permits a rapid, genus-wide detection of legionella antigens in liquids of the aforementioned type, particularly in the urine, with a high specificity, high sensitivity, whilst the process can be performed in a simple and rapid manner using readily accessible starting materials.

EP 0 364 971 A2

**Process for the detection of legionella antigens in body fluids and the like, as well as a test kit for performing the process.**

The invention relates to a process for the detection of legionella antibodies in body fluids, tissue homogenates or in other legionella antigen-containing fluids with specific antibodies for this, accompanied by the formation of a labelled antigen-antibody complex and its detection by standard immunoassays. It also relates to a particularly suitable test kit for performing this process.

Since legionellosis was first described, there has been an urgent need for a rapid diagnostic process for detecting a legionella disease. Processes for the detection of legionella antigens have already been proposed, but suffered from the limitation that they only covered a narrow range of germs and could not be commercially developed as a result of technical problems. The fundamental problem was inter alia that no antibodies could be produced, which could detect in "genus-wide" manner "in vivo processed" antigen (e.g. urine antigen).

Processes of the aforementioned type are known, e.g. from the literature references Zentralbl. Bakteriol. Mikrobiol. Hyg. (A) 1983, 225: 102-107, J. Clin. Micriobiol. 1984, 20: 605-607, J. Infect. Dis. 1984, 152: 1007-1012, Ann. Microbiol. (Paris), 1983, 134A: 155-161, Am. J. Med. 1982, 72: 576-582 and J. Clin. Microbiol. 1982, 16: 1007-1011. It is unimportant in which type of fluids the legionella antigens occur and can be detected, i.e. they can be body fluids, such as sputum, tracheal aspirations, serum, liquor, pleural fluid or tissue homogenates, e.g. from the lung, heart or tissues of other organs. Fundamentally the process also permits the detection of legionella antigens in potentially legionella-containing liquids, such as drinking water, surface water, industrial waste water and sewage, technically used water for cooling or moistening purposes or water used in other ways from water plants.

Numerous working groups have in the past developed immunoassays, which, on the basis of polyclonal or monoclonal antibodies, aimed at detecting legionella antigens in body fluids. In antibody extraction or isolation, these processes are always based on the bacterial cell used either whole or in disintegrated form for extracting the antibody fractions. The thus obtained antibodies suffer from the disadvantage that they frequently did not detect the processed legionella antigens present in body fluids. They were also unsuitable for ensuring a genus-wide detection of the most varied legionella bacteria. Consideration was not given to the fact that processed antigens frequently only have a few antigen-binding points and carry preserved and also new antigen determinants. It was particularly important in establishing a new test system to detect all legionella types potentially having a pathogenic effect in humans by means of a universal test. Thus, there was no test making it possible to detect in genus-wide manner legionella antigens in the urine.

The problem of the invention was therefore to develop a test extending over and beyond the previously known processes so as to enable it to be used more widely and detect all previously known human pathogenic legionellas or their processed antigens in fluids of the aforementioned type.

According to the invention, this problem is solved in that the immunoassay is mainly directed at the detection of an in vivo or in vitro processed antigen fraction of an apparent molecular weight of approximately 68 and/or approximately 25 kDa, determined by the SDS-PAGE method.

The present invention was developed in the following way. Firstly the legionella antigens were characterized in the urine, the genus-wide cross-reacting antigens being identified by the Western blot method. It was possible to show that mainly two molecular weight fractions in the range approximately 68 and 25 kDa (determined by the SDS-PAGE process) cross-react in genus-wide manner. Therefore these fractions could be looked upon as a common antigen to all species. Thus, this research provided the new finding that there is a common antigen, not found in previous research, with a molecular weight of approximately 68 kDa in the urine, which can apparently not be detected in the disintegrated bacterial cell. It was simultaneously revealed for the first time that during human legionellosis a large number of different antigens can be detected in the urine by means of the immunoblot method.

Although the 25 and 68 kDa antigen fractions have the greatest significance for the test in the genus-wide detection of processed legionella antigens, the other urine antigen fractions in the molecular weight range of approximately 100 to 10 kDa identified, characterized and partly processed by us for the first time were taken into account in creating an appropriate capture antibody. It was ensured in this connection (immunoblot process), that the "vaccine" used for producing the capture antibody had a very marked immune response, also with respect to the remaining urine antigens of the fractions (100 to 10 kDa). These cross-reacting antibodies, which although not genus-wide are in part "interspecies-wide", as proved by tests, significantly increase the sensitivity of the test.

Legionella antigens can also be detected in other body fluids and the like according to the present invention, such as in the blood, sera, tissue homogenate and the like. The immunoblot tests also revealed that unprocessed antigen, such as is available in bacterial cell disintegrations or in research materials from other bacterial sources of legionella, such as e.g. environmental specimens, swimming bath water or other liquids defined hereinbefore, can be detected according to the invention.

Thus, the essence of the present invention is the finding that the aforementioned antigen fractions with an apparent molecular weight of approximately 68 and/or 25 kDa permit the genus-wide detection of legionella antigens. The resultant advantages will be studied hereinafter. There is no limitation to a specific immunoassay for realizing the inventive concept. On the basis of his general knowledge, the Expert is in a position to choose the most suitable immunoassay for the particular case. Thus, it can e.g. be a competitive immunoassay, but also the so-called sandwich assays. In general, preference is given to a sandwich assay. The following explanations largely relate to such an assay. However, to the extent possible, they should also apply for other immunoassays. The labelling or markng question is also not of critical importance. It places no significant restrictions on the manufacturer of e.g. the antibody conjugate in the case of the inventive process. Thus, labelling can take place with radioisotopes (RIA), with enzymes (ELISA) and with fluorescence markers or any other chromophoric group. Preference is given to enzyme immunoassay (ELISA). Preferably peroxidase or alkaline phosphatase is used as the enzyme.

Various ways can be adopted for obtaining the antibody conjugate and the capture antibody to be used for the sandwich assays. Thus, e.g. the capture antibody can be directed at an extensive detection of the processed antibody fractions in the test material, whilst the antibody conjugate mainly has a particular specificity with respect to the processed antigen fractions of the apparent molecular weight approximately 68 and/or 25 kDa. Fundamentally, the reverse procedure is also possible, i.e. the particular specificity exists with regards to the capture antibody, whereas the antibody conjugate can be largely deposited on all still free epitopes of the processed antigen fraction bound to the capture antibody. As the "capture" the antibody should be such that it fulfils its capture function in an optimum way. It must be borne in mind that it frees sufficient epitope for the antibody conjugate to be bound to the desired extent. For this purpose it is preferable for the capture antibody and the antibody conjugate to have different specificities. Bearing in mind this basic principle, the inventive process is optimized in that an antibody conjugate is used, which binds in genus-wide manner legionella antigens and to this extent is complimentary to the capture antibody used and also binds to still free epitope of the legionella antigen bound from the capture antibody. Obviously extensive modifications are possible. However, preference is given to the first-mentioned variant. In order to achieve an optimization, the antibodies should have different specificity. Detailed comments thereon are provided hereinafter.

Fundamentally the process of the invention can be carried out with both monoclonal and polyclonal antibodies. However, experience has shown that preference is given to polyclonal antibodies within the scope of the standard immunoassays used in this test. The polyclonal antibodies used not only ensure the binding of the specifically defined antigen fractions, but also the binding of a large number of other antigen fractions detected in the urine and therefore constitute a significantly widened basis for the binding of antigen, so that detection sensitivity is increased.

The following procedure can be adopted for producing the desired antibody combination (capture antibody/antibody conjugate).

Firstly those legionella antigen fractions are selected, which permit an optimum detection of urine antigens in the Western blot process. These antigen fractions are used in the conventional way for immunizing test animals and the effectiveness of an antibody formation is again tested in the immunoblot process. Immunoglobulins, particularly the IgG fraction, are obtained from the hyperimmune serum of animals using standard separating processes. The test animals are immunized with the indicated different antigen fractions. In order to isolate the antiserum from which the conjugate is prepared, preference is given to the use of the 68 kDa antigen fraction. For isolating the capture antibody fraction, use is made in a selection process for immunization of those antigen fractions which have proved to be particularly effective in immunoblot analysis for detecting legionella urine antigens. Detailed comments are provided in the following example on the advantageous procedure adopted.

For the detection of the labelled antigen-antibody complex formed in the process according to the invention, it is unimportant for the reaction on solid phases which of the antibodies obtained is used as the capture or the conjugate and immobilized. Preferably the capture antibody is immobilized. The word "immobilized" is to be understood in its widest sense. Thus, the capture antibody can be immobilized on a plastic surface or random other carrier materials. No significant part is played by the external configuration of the solid phase. These can be planar surfaces, but also dispersed spherules or textile materials. This list is not intended to constitute any limitation. The advantage of immobilizing the capture antibody is that the following identification reaction takes place at a clearly defined point.

The antibody conjugate preferably has a specificity with respect to a genus-wide antigen fraction with an apparent molecular weight of approximately 68 kDa. This antibody can fundamentally be used for detection in a single or double sandwich method. For the purposes of the inventive process, preference is given to the use of the single sandwich method, because there are less possibilities of error.

The following special advantages result from the invention. The advantage of the test is the rapid antigen detection, which can be carried out with great sensitivity, specificity and conventional technical equipment, conveniently in the physical format described in EP 291 194.7. It is suitable for wide use and the genus-wide detection of legionella antigens in body fluids, tissue homogenates and other test materials. Fundamentally the test can be carried out either as a plate test according to the standard ELISA method, or in a simple dipping process by means of a capture antibody fixed to surfaces. Thus, the presently developed test fulfils the requirements which have hitherto been made. It can be transferred to the format of a commercial test and on a genus-wide basis covers all the hitherto known pathogenic legionella species in man and satisfies the demand for a rapid diagnostic process.

The invention is further illustrated by the following example.

Example

a) ELISA for detecting the legionella urine antigen.

Biochemical and immunological characterization of the urine antigen of different legionella species gave "genus-wide" antigen common points. The urine antigen of Lp (serum group I) was purified and used for immunizing rabbits. Using this rabbit hyperimmune serum cross-reacting antigen bands (urine) were determined with the immunoblot method and their apparent molecular weights determined. It could be gathered from the biochemical analysis of cross-reacting (urine) antigens of the molecular weight ranges 20 to 30 and 50 to 70 kDa, that they came from the decomposition of the bacterial cell sleeve. Further research for developing an ELISA for the early detection of legionella antigens was concentrated on two points:

1) The preparation of a vaccine (immunization of rabbits), inducing high specificity antibodies for legionella urine antigen (68/25 kDa) with a genus-wide reactivity.

2) Standardization of an immunization process for preparing a high-titre antiserum in the rabbit and for transferring this system to the ram.

b) Preparation of the vaccine.

The bacterial suspension of L pneumophila, serum group I, was disintegrated in a disintegrator (AEG, type DM 68/4-2) with glass beads (diameter 0.17-0.18 mm) - B. Braun Melsungen AG, for 4 minutes and with $CO_2$ cooling. After autoclaving (20 min, 120°C, 1 atm overpressure), the cell sleeve formed from cytoplamic membrane (CM) peptidoglycan (PG) and the outer membrane (OM), was obtained by centrifuging. For immunization, the cell sleeve was so resuspended in PBS, that 1 ml of the suspension contained 2 mg of protein - vaccine 1. The cell sleeve was fractionated according to the following diagram for the further immunization tests:

**Cell sleeve**

(CM, PG, OM) Vaccine I

0.05 M tris-HCL, pH 9.0; 1% Triton X-100
shaken for 2 h at room temperature

centrifuged for 90 min, 20,000 rpm

Supernatant cytoplasmic
membrane (CM)

Vaccine ②

Protein: 2 mg/ml

Sediment-peptidoglycan (PG)
outer membrane (OM)

0.05 M tris-HCL pH 9.0;

0.01 M EDTA; 0.5% SDS

shaken for 2 h at room
temperature

centrifuged 90 min, 20,000 rpm

Supernatant – outer
membrane (OM)

Vaccine ③

Protein: 2 mg/ml

Sediment – pepti-
doglycan (PG)

Vaccine 4
Suspension:
$OD_{660} = 1.120$

c) Preparation of antisera and use in ELISA

In each case 0.5 ml of the prepared "vaccines" (I-4) were mixed 1:1 with the complete Freund's adjuvant and administered subcutaneously (s.c.) and intramuscularly (i.m.) to different points of the back of the rabbit. On the seventh day, the animals received a further 1 ml of vaccine i.m. The following immunizations took place with the same quantity of antigen in 0.5 ml of PBS six weeks after the primary immunization. On three successive days the antigen of day 1 was injected i.m. into the thigh and on the second and third days i.v. into the ear vein. After one week the same immunization program was repeated. Blood was withdrawn fourteen days after the final immunization.

The immunization was followed up on a weekly basis, following the withdrawal of blood from the ear veins, by means of the urine antigen band pattern detected by the immunoblot process. Particular importance was attached to the determination of a reactivity against the 25 and 68 kDa antigens, which could be detected in all the investigated positive urines with antisera of "vaccine" I from the rabbit and ram. IgG fractions were obtained by ion exchange chromatography from the antisera against the "cell vaccine" and against the purified and fractionated urine antigen of Lp SG-I and peroxidase conjugate was prepared. For this purpose all IgG preparations were chromatographed against the urine antigen over a human serum agarose column, in order to eliminate any possible specificity against human serum proteins.

Various combinations of capture and conjugate were tested for sensitivity and genus-wide detection of legionella urine antigens using the so-called "antibody sandwich assay".

In order to test the genus-wide detection of antigens, use was made of guinea pig urine following intraperitoneal infection with different legionella species, as well as human urine of patients found to have legionellosis by culture detection. The maximum sensitivity and genus-wide detection were obtained with the antibodies against vaccine I (as capture) and the antibodies against 68 kDa urine antigen (as conjugate). This combination gave a low cross-reactivity with 10 x concentrated guinea pig urine following intraperitoneal infection with E. coli, Ps aeruginosa, Ps. fluoresces and B. pertussis, but this could be eliminated by absorption of the capture with E. coli. Only absorbed sera was used in the final test.

## Claims

1. Process for the detection of legionella antigens in body fluids, tissue homogenates or in other legionella antigen-containing liquids with specific antibodies for the same, accompanied by the formation of a labelled antigen - antibody complex and its detection by means of standard immunoassays, characterized in that the immunoassay is mainly directed at detecting an in vivo or in vitro processed antigen fraction with an apparent molecular weight of approximately 68 and/or approximately 25 kDa, determined by the SDS-PAGE method.

2. Process according to claim 1, characterized in that at least two antibodies of different specificity are used for forming the labelled antigen - antibody complex.

3. Process according to claims 1 or 2, characterized in that legionella antigens from the urine are detected.

4. Process according to claims 1,2 or 3, characterized in that an immunoassay is performed with polyclonal antibodies.

5. Process according to claims 3 or 4, characterized in that polyclonal "capture" antibodies with an excellent specificity with respect to legionella urine antigens are used.

6. Process according to at least one of the claims 1 to 5, characterized in that for forming the antigen-antibody complex, firstly an immobilized capture antibody is used.

7. Process according to at least one of the claims 1 to 6, characterized in that for forming the complex an antibody conjugate is used, which has a different specificity to the capture antibody.

8. Process according to at least one of the claims 1 to 7, characterized in that an enzyme immunoassay is performed.

9. Process according to claim 8, characterized in that an enzyme immunoassay is performed with peroxidase or alkaline phosphatase.

10. Process according to at least one of the claims 1 to 9, characterized in that an antibody conjugate is used, which binds legionella antigens in genus-wide manner and to this extent is complimentary to the "capture" antibody used and therefore binds still free epitope of the legionella antigen bound to the capture antibody.

11. Process according to at least one of the claims 1 to 10, characterized in that an antibody conjugate is used having specificity with respect to an antigen fraction with an apparent molecular weight of approximately 68 kDa.

12. Test kit for performing the process according to at least one of the claims 1 to 11, characterized in that it comprises a) an antibody conjugate and b) a capture antibody, at least one antibody being mainly directed at the detection of an in vivo or in vitro processed antigen fraction with an apparent molecular weight of approximately 68 and/or approximately 25 kDa, determined by the SDS-PAGE method.